# EUROPÄISCHE PATENTSCHRIFT

(11) **EP 2 118 296 B1**
(45) Veröffentlichungstag und Bekanntmachung des Hinweises auf die Patenterteilung: **11.05.2016**
(21) Anmeldenummer: 08717534.5
(22) Anmeldetag: 07.03.2008
(51) Int. Cl.: C09J 103/02, D21H 17/00, D21H 17/28, C08B 30/12, C08B 30/16, C12P 19/14

(54) **VERFAHREN ZUR KONTINUIERLICHEN HERSTELLUNG VON ABGEBAUTEM STÄRKEKLEISTER UND ANLAGE ZUR DURCHFÜHRUNG EINES SOLCHEN VERFAHRENS**
METHOD FOR THE CONTINUOUS PRODUCTION OF DEGRADED STARCH PASTE AND SYSTEM FOR PERFORMING SUCH A METHOD
PROCÉDÉ POUR LA PRODUCTION CONTINUE DE COLLE D'AMIDON DÉGRADÉE ET INSTALLATION POUR LA MISE EN UVRE D'UN TEL PROCÉDÉ

(30) Priorität: 08.03.2007 DE 102007011409
(43) Veröffentlichungstag der Anmeldung: 18.11.2009
(73) Patentinhaber: BVG Bauer Verfahrenstechnik GmbH, 86926 Greifenberg (DE)
(72) Erfinder: BAUER, Jochen, 86922 Eresing/Pflaumdorf (DE)
(74) Vertreter: Leske, Thomas
(86) Internationale Anmeldenummer: PCT/EP2008/052787
(87) Internationale Veröffentlichungsnummer: WO 2008/107485

(56) Entgegenhaltungen:
- EP-A- 1 386 960
- CH-A- 513 980
- DE-A1- 1 567 360
- US-A- 3 133 836
- US-A- 3 404 071
- US-A- 4 014 743
- US-A- 4 957 563

## Beschreibung

Die Erfindung betrifft ein Verfahren zur kontinuierlichen Herstellung von Stärkekleister aus enzymatisch abgebauter Stärke, welcher beispielsweise in der Papierindustrie eingesetzt wird, sowie eine Anlage zur Durchführung eines solchen Verfahrens.

Die kontinuierliche Herstellung von enzymatisch abgebautem Stärkekleister wird üblicherweise in einem sogenannten "Plugflow"-Verfahren durchgeführt. In diesem Verfahren, welches auch als Pfropfen- oder Kolbenströmungsverfahren bekannt ist, wird Stärkesuspension durch Erhitzen verkleistert, mit Stärke-abbauenden Enzymen (Amylasen) versetzt und kontinuierlich durch einen entsprechenden "Plugflow"-Konverter geleitet, in welchem der enzymatische Abbau der Stärkemoleküle stattfindet.

Ziel des "Plugflow"-Verfahrens ist es, eine gleiche Verweilzeit für jedes Stärkemolekül im "Plugflow"-Konverter zu erreichen. Es wird versucht, die Stärke kontinuierlich in Schichten durch den Konverter zu führen, um eine gleiche Verweilzeit aller Stärkemoleküle im Konverter sicherzustellen. Damit die nötige Schichtenströmung erzeugt werden kann, wird im "Plugflow"-Konverter auf ein Rührwerk weitgehend verzichtet. Weiterhin werden, um die Bildung von Turbulenzen im Konverter zu verhindern, in der Regel Siebbleche in den Konverter eingebaut.

Allgemeine Lehrmeinung ist, dass bei der Herstellung von abgebautem Stärkekleister im kontinuierlichen Betrieb eine gleiche Verweilzeit für jedes Stärkemolekül im Konverter erreicht werden muss, um aufgrund der Zeitabhängigkeit enzymatischer Umsetzungsreaktionen jedes Stärkemolekül gleichmäßig dem enzymatischen Abbau auszusetzen. Nur unter Berücksichtigung dieser Grundvoraussetzung können nach herrschender Meinung die für den abgebauten Stärkekleister gewünschten Eigenschaften erreicht werden.

Nachteilig am Stand der Technik ist, dass aufgrund der gleichen Einwirkzeit der Enzyme auf die Stärkemoleküle im "Plugflow"-Konverter alle Stärkemoleküle den gleichen Grad an enzymatischen Abbau erfahren, was eine Verschlechterung der Eigenschaften des abgebauten Stärkekleisters zur Folge hat.

Weiterhin nachteilig am Stand der Technik ist, dass durch die auf alle Stärkemoleküle gleich lang einwirkende Aktivität der Amylasen ein hoher Glukosegehalt in dem abgebauten Stärkekleister erzeugt wird, welcher die Klebkraft des abgebauten Stärkekleisters reduziert.

Nachteilig ist weiterhin, dass aufgrund der Vermeidung von Turbulenzen im "Plugflow"-Konverter der Stärkekleister und die Enzyme während der Reaktion nur ungenügend miteinander vermischt werden. Dies beeinträchtigt die Effizienz der Abbaureaktion und macht den Einsatz von größeren Mengen von abbauenden Enzymen nötig.

Die in den "Plugflow"-Verfahren erwünschte Vermeidung von Turbulenzen innerhalb des Konverters führt weiterhin zu verstärkten Ablagerungen des Stärkekleisters in Bereichen des Konverters, in denen keine oder nur sehr geringe Strömungen auftreten. Diese sogenannte Trombenbildung führt zu einer erhöhten Heterogenität des Stärkekleisters und kann Verstopfungen von kleineren Öffnungen, zum Beispiel innerhalb von Ventilen, führen. Weiterhin können Ablagerungen, die sich an Messgliedern im Konverter bilden, zu ungenauen Messwerten führen und so die Erzeugung einer gleichbleibenden Kleisterqualität erschweren.

Das Auftreten von Ablagerungen in "Plugflow"-Konvertern hat ferner zur Folge, dass die Konverter öfter und intensiver gereinigt werden müssen. Dies verursacht durch den erhöhten Abwasserverbrauch und den Stillstand des Konverters während der Reinigungsphasen zusätzliche Kosten, welche die Wirtschaftlichkeit des Verfahrens beeinträchtigen.

US 4,014,743 beschreibt ein kontinuierliches Verfahren zur Herstellung von Stärkepaste, bei dem getrennt voneinander eine wässrige Stärkesuspension und eine Enzymlösung in einen Rührkessel gegeben und dort verrührt werden. Der Rührkessel weist ein Rührwerk auf und wird durch Dampf beheizt. Die abgebaute Stärke verlässt den Kessel am unteren Ende. In dem Maß, in dem das Produkt den Kessel verlässt, werden die Edukte (Stärkesuspension und Enzymlösung) nachgegeben. Die Reaktion wird durch Messung der Viskosität des Produktes überwacht. Das Enzym (in der Produktlösung) wird nach dem Verlassen des Kessels inaktiviert.

Aus WO 00/11957 bekommt der Fachmann den Hinweis, dass eine Verkleisterung und komplette Hydrolyse der eingesetzten Stärke, welche in dem Verfahren ebenfalls ausschließlich in einem Rührkessel unter Zusatz von Wasser und Enzymen aufbereitet wird, im Normalfalle etwa eine Stunde dauert. Dabei ist das Verfahren ein sogenanntes Plugflow-Verfahren, das heißt die zugegebenen und abgegebenen Eingans- bzw. Ausgangsstoffe fließen nicht kontinuierlich, sondern in einer Art "Pfropfenströmung".

DE 37 31 293 A1 rühmt sich speziell damit, dass es mit dem Verfahren möglich ist, die Endkonzentration an Stärke in der Stärkepaste auf einen Feststoffgehalt von bis zu 70% an Stärke, gemessen in der Trockensubstanz, anzuheben. Hieraus ist ersichtlich, dass die allgemeine Lehre für die Herstellung von Kleister bisher davon ausgegangen ist, dass je mehr Stärke in dem Kleister verarbeitet wird, desto stärker werden dessen Eigenschaften verbessert. Dabei wird in Kauf genommen, dass bei dieser Art der Herstellung von Kleister zum Erreichen einer gewünschten Klebkraft eine lange Verweilzeit der Stärkemoleküle, die separat von den Enzymen zugeführt werden, in dem Rührkessel benötigt wird. Hieraus lässt sich direkt entnehmen, dass in dem Rührkessel eine Schichtung der Stärkesuspension mit Enzymen von oben nach unten aufgebaut wird, wobei die Verweilzeit von oben nach unten zunimmt.

DE 21 59 315, welches ein diskontinuierliches Verfahren zur Stärkekleisterherstellung mit Verkleisterung in einem Reaktionsraum beschreibt, enthält keinen Hinweis, wie eine Verweilzeit bzw. Einwirkdauer soweit reduziert werden kann, dass die eingesetzten Edukte innerhalb kürzester Zeit als Kleister verwendet werden können. DE 21 59 315 geht über ein herkömmliches Plugflow-Verfahren zur Erhöhung der Stärkekonzentration im Kleister nicht hinaus und bietet dem Fachmann somit keinerlei weitere Hinweise.

Die Auslegeschrift DT 1 243 120 zeigt eine Anlage, in der eine quasikontinuierliche StärkeAbbau-Reaktion ausschließlich in einem Reaktor stattfindet. Zwar ist es hier prinzipiell möglich sowohl die Eingangsstoffe kontinuierlich zuzuführen als auch das Endprodukt kontinuierlich zu entnehmen. Jedoch sind bei diesem Verfahren, wie in allen herkömmlichen Verfahren üblich, Stärkeabbau-Puffer vorgesehen, welche es der Stärke bzw. den einzelnen Stärkemolekülen ermöglichen, eine gewisse Verweilzeit, hier bevorzugt die Reaktionszeit, innerhalb des Reaktors der zu verbleiben.

Aufgabe der Erfindung ist es, ein gegenüber dem Stand der Technik verbessertes Verfahren zur kontinuierlichen Herstellung von abgebautem Stärkekleister bereitzustellen, mit welchem abgebauter Stärkekleister erzeugt wird, der in seinen Eigenschaften gegenüber dem mit bekannten Verfahren hergestellten abgebauten Stärkekleister verbessert ist. Weiterhin ist es Aufgabe der Erfindung eine Anlage zur Durchführung dieses verbesserten Verfahrens bereitzustellen.

Diese Aufgabe wird durch ein Verfahren mit den Merkmalen gemäß Anspruch 1 und durch eine Anlage zur Durchführung dieses Verfahrens mit den Merkmalen gemäß Anspruch 9 gelöst. Zweckmäßige Weiterbildungen der Erfindung sind in den jeweiligen abhängigen Ansprüchen definiert.

In dem erfindungsgemäßen Verfahren zur kontinuierlichen Herstellung von enzymatisch abgebautem Stärkekleister werden einem Rührkessel, in welchem ein enzymatischer Abbau stattfindet, Stärkemoleküle enthaltende Stärkesuspension und zumindest zeitweise Enzym zugeführt und von dem Rührkessel gleichzeitig abgebauter Stärkekleister kontinuierlich abgeführt. Dabei wird im Rührkessel ein so stark turbulenter Zustand erzeugt, dass selbst bei kürzester Verweilzeit einzelner Stärkemoleküle deren den gewünschten Eigenschaften des Stärkekleisters entsprechender Abbau stattfindet.

Unter kürzester Verweilzeit ist dabei das Zeitintervall zu verstehen, welches ein Stärkemolekül benötigt, um von der Stelle des Eintritts in den Rührkessel unter Zurücklegung des kürzesten Wegs die Stelle des Austritts aus dem Rührkessel zu erreichen.

Unter den durch das erfindungsgemäße Verfahren hervorgerufenen gewünschten Eigenschaften des Stärkekleisters sind die Eigenschaften des abgebauten Stärkekleisters zu verstehen, welche durch physikalische und molekulare Beschaffenheit des Stärkekleisters bestimmt werden. Bevorzugt als gewünschte Eigenschaften ist das Aufweisen einer hohen Klebkraft. Weiterhin bevorzugt, als gewünschte Eigenschaft, ist eine gute Verarbeitbarkeit oder eine gute Lagerungsfähigkeit. Weiterhin bevorzugt, als gewünschte Eigenschaften sind Kombinationen aus hoher Klebkraft, guter Verarbeitbarkeit und guter Lagerungsfähigkeit.

Der stark turbulente Zustand, der nach dem erfindungsgemäßen Verfahren im Rührkessel erzeugt wird, bewirkt eine optimale Vermischung des zugegebenen Enzyms mit dem Stärkekleister. Um diesen stark turbulenten Zustand zu erzeugen, wird ein Reaktionsgefäß verwendet, welches als ein Rührkessel ausgebildet ist.

Bevorzugt kommen die Eigenschaften des Rührkessels den Eigenschaften eines idealen Rührkessels nahe. In einem idealen Rührkessel findet eine vollständige Vermischung der Edukte und der durch die Reaktion der Edukte entstehenden Produkte statt. In einem idealen Rührkessel werden die zufließenden Edukte mit unendlich hoher Geschwindigkeit mit der Masse im Reaktionsraum des Rührkessels vermischt. Da im Rührkessel durch die Umsetzung der Edukte und die damit einhergehende Entstehung von Produkten eine geringere Eduktkonzentration als in der Zulaufleitung herrscht, liegt an der Eintrittsstelle in den Rührkessel ein Konzentrationssprung vor. Dies bedeutet, dass in einem idealen Rührkessel die Konzentration der Edukte, welche in den Kessel eintreten, sofort bei Kesseleintritt auf die im Kessel herrschende niedrigere Konzentration absinkt.

Weiterhin ist die Konzentration aller Stoffe im idealen Rührkessel zeitlich und örtlich gleich. Dabei haben die in den Rührkessel eintretenden Stärkemoleküle keine gleiche Verweilzeit im Rührkessel, sondern eine mittlere Verweilzeit. Einzelne Stärkemoleküle können sehr lange und andere Stärkemoleküle können eine sehr kurze Verweilzeit im Rührkessel haben.

Bevorzugt beträgt in dem erfindungsgemäßen Verfahren die Verweilzeit eines Stärkemoleküls im Rührkessel eine Sekunde (1 s) bis zu einer Stunde (1 h).

Das nach dem erfindungsgemäßen Verfahren die Stärkemoleküle abbauende Enzym ist bevorzugt eine Amylase. Amylasen sind Hydrolasen, welche die Stärkemoleküle an der glykosidischen Bindung hydrolytisch spalten. Amylasen werden auch als Saccharidasen, also als Enzyme, welche Polysaccharide spalten, eingestuft. Weiterhin bevorzugt ist das bei dem erfindungsgemäßen Verfahren verwendete Enzym eine alpha-Amylase. Bevorzugt ist auch der Einsatz von beta-Amylasen. Weiterhin bevorzugt ist eine Kombination aus alpha- und beta-Amylasen.

Bevorzugt ist die Verwendung von nativen oder modifizierten Amylasen. Als native Amylasen werden Amylasen erachtet, die aus natürlich vorkommenden Organismen gewonnen werden oder deren molekulare Eigenschaften, wie zum Beispiel die Aminosäuresequenz, den molekularen Eigenschaften von aus natürlich vorkommenden Organismen gewonnenen Amylasen entsprechen. Als modifizierte Amylasen werden Amylasen erachtet, welche, beispielsweise durch gentechnische Methoden, in ihren molekularen Eigenschaften, wie zum Beispiel ihrer Aminosäuresequenz, verändert wurden. Bevorzugt sind Amylasen, welche ein Reaktionsoptimum bei 75-90°C haben. Weiterhin bevorzugt sind Amylasen, welche durch eine Erhöhung der Temperatur bis zu 75-90°C aktiviert werden. Bevorzugt beträgt der Enzymanteil im erfindungsgemäßen Verfahren - bezogen auf die Stärke - 0,02% - 0,2%.

Als alternative Ausführungsform wird in dem erfindungsgemäßen Verfahren bevorzugt zusätzlich zu dem Stärke-abbauenden Enzym ein oder werden mehrere weitere Polymerabbauende Enzyme der Stärkesuspension beigefügt. Bevorzugte weitere Enzyme sind Ligninasen, Cellulasen, Hemicellulasen, Xylanasen oder Proteasen.

Die bei dem erfindungsgemäßen Verfahren in der Stärkesuspension enthaltenen Stärkemoleküle sind bevorzugt native oder modifizierte Stärkemoleküle. Native Stärke besteht zu 10 - 30 % aus Amylose (lineare Ketten von ausschließlich alpha-1,4-glykosidisch verknüpften Glukosemolekülen) und zu 70 - 90 % aus Amylopektin (stark verzweigte Ketten mit alpha-1,6-glykosidisch und alpha-1,4-glykosidisch verknüpften Glukosemolekülen). Die native Stärke ist bevorzugt Kartoffelstärke. In einer alternativen Ausführungsform ist die native Stärke bevorzugt Weizenstärke, Erbsenstärke, Reisstärke, Maisstärke oder Tapiokastärke. Weiterhin wird bevorzugt eine Mischung aus verschiedenen nativen Stärkesorten verwendet.

In einer alternativen Ausführungsform wird bevorzugt modifizierte Stärke verwendet. Unter modifizierter Stärke ist native Stärke zu verstehen, welche chemisch, physikalisch oder enzymatisch verändert wurde. Bevorzugte modifizierte Stärke ist mit Säure behandelte Stärke, mit Laugen behandelte Stärke, mit Bleichmitteln, wie zum Beispiel Peroxyessigsäure, Wasserstoffperoxid, Natriumhypochlorit, Natriumchlorit, Schwefeldioxid, Sulfiten, Kaliumpermanganat oder Ammoniumpersulfat, gebleichte Stärke, enzymatisch modifizierte Stärke, durch Oxidation modifizierte Stärke, durch Veresterung modifizierte Stärke, veretherte Stärke, kationische Stärke und durch Phosphorylierung modifizierte Stärke. Weiterhin bevorzugt ist die Verwendung von durch Reaktion mit Propylenoxid oder durch Acetylierung modifizierte Stärke. Bevorzugt ist ferner die Verwendung einer Mischung aus nativer und modifizierter Stärke.

Die in dem erfindungsgemäßen Verfahren zugeführte Stärkesuspension wird vor dem Eintritt in den Rührkessel oder im Rührkessel verkleistert. Bevorzugt wird die Verkleisterung durch Erhöhung der Temperatur erreicht. Bei der Verkleisterung durch Erhöhung der Temperatur bindet die Stärke durch die Hitzeeinwirkung ein Vielfaches ihres Eigengewichtes an Wasser und quillt dadurch auf. Die zur Verkleisterung der Stärkesuspension verwendete Temperatur ist bevorzugt 65-90°C.

In einer alternativen Ausführungsform kann die Stärkesuspension bevorzugt durch Zugabe von Laugen verkleistert werden. Bevorzugt kann dabei der erhöhte pH-Wert im Anschluss an die Verkleisterung durch Zugabe von Säuren wieder auf den für das abbauende Enzym optimalen pH-Wert eingestellt werden.

In dem erfindungsgemäßen Verfahren wird in dem abgeführten Stärkekleister befindliches Enzym inaktiviert. Die Inaktivierung erfolgt bevorzugt durch Erhöhung der Temperatur. Die Inaktivierungstemperatur beträgt bevorzugt 100 - 140°C.

In einer alternativen Ausführungsform kann die Inaktivierung des in dem abgeführten Stärkenkleister befindlichen Enzyms bevorzugt durch Zugabe eines Enzym-Inhibitors inaktiviert werden. Bevorzugte Enzym-Inhibitoren sind aus Pflanzen gewonnene Enzym-Inhibitoren. Weiterhin bevorzugt sind gentechnisch hergestellt Enzym-Inhibitoren oder alternativ bevorzugt synthetische Inhibitoren. Enzym-Inhibitoren sind bevorzugt Proteine oder alternativ bevorzugt andere organische Verbindungen.

In einer weiteren alternativen Ausführungsform wird das Enzym durch eine Veränderung des pH-Werts inaktiviert.

Das erfindungsgemäße Verfahren zur kontinuierlichen Herstellung von enzymatisch abgebautem Stärkekleister ist in der erfindungsgemäßen Anlage durchführbar, welche mindestens eine Zuleitung zum Einbringen der Stärkesuspension und des Enzyms in einen Rührkessel, ein Modul zur Verkleisterung der Stärkesuspension, den Rührkessel mit einem Rührwerk zur Erzeugung eines turbulenten Zustands darin, eine Steuereinheit, mittels welcher erste Signale zur Regulierung des Zustands im Rührkessel erzeugbar sind, mindestens eine Ableitung zur insbesondere kontinuierlichen Abführung des abgebauten Stärkekleisters aus dem Rührkessel und Stellglieder, welche mittels durch die Steuereinheit erzeugter zweiter Signale regelbar sind, aufweist.

Bevorzugt werden die Stärkesuspension und das Enzym gemeinsam über eine Zuleitung in den Rührkessel eingebracht. Bevorzugt werden dabei das Enzym und die Stärkesuspension vor der Verkleisterung der Stärkesuspension vermischt. In einer alternativen Ausführungsform erfolgt die Zugabe des Enzyms zur Stärkesuspension nach der Verkleisterung der Stärkesuspension. Die Verkleisterung der Stärkesuspension erfolgt bevorzugt vor dem Eintritt in den Rührkessel. In einer alternativen nicht vom Erfindungsgedanken umfassten Ausführungsform erfolgt die Verkleisterung im Rührkessel.

Die Vorrichtung zur Erzeugung eines turbulenten Zustands im Rührkessel ist ein Rührwerk, welches bevorzugt über einen Motor angetrieben wird, der durch den Erhalt von Signalen aus der Steuereinheit regulierbar ist. Weiterhin bevorzugt verfügt der Rührkessel über Schikanen oder andere Strömungsbrecher, welche geeignet sind, den turbulenten Zustand im Rührkessel zu verstärken und so eine noch größere Durchmischung von Stärkesuspension und Enzymen zu erreichen. Der Rührkessel verfügt über ein Rührwerk und bevorzugt zusätzliche Schikanen oder andere Strömungsbrecher. Das Reaktionsgefäß ist ein Rührkessel, dessen Eigenschaften bevorzugt den Eigenschaften eines idealen Rührkessels nahe kommen.

Das Reaktionsgefäß ist als ein Rührkessel ausgebildet, welcher mit einem Rührwerk ausgestattet ist, dessen Rührgeschwindigkeit über die Signale der Steuereinheit regulierbar ist, wodurch der turbulente Zustand im Reaktionsgefäß regulierbar ist.

Weiterhin bevorzugte regulierbare Zustände im Rührkessel sind die Temperatur und der pH-Wert der das Enzym enthaltenden Stärkesuspension, die Stärkesuspensionskonzentration und die Enzymkonzentration. Bevorzugt ist die erfindungsgemäße Anlage so ausgestaltet, dass ein oder mehrere unterschiedliche Zustände, ausgewählt aus der Gruppe bestehend aus turbulenter Zustand, Temperatur, pH-Wert, Stärkesuspensions- und Enzymkonzentration der Enzym enthaltenen Stärkesuspension im Rührkessel durch die Steuereinheit reguliert werden.

Zur Einstellung des pH-Werts verfügt die erfindungsgemäße Anlage in einer bevorzugten Ausführungsform über eine oder mehrere weitere regulierbare Zuleitungen, durch welche Säuren und Laugen zusammen oder getrennt voneinander in den Rührkessel eingeleitet werden.

Die Stellglieder der erfindungsgemäßen Anlage sind bevorzugt in der Lage, die Zufuhr der Stärkesuspension und des Enzyms, die Verkleisterung der Stärkesuspension und die Abführung des abgebauten Stärkekleisters zu regulieren. Bevorzugt werden die Stellglieder von der Steuereinheit reguliert. Weiterhin bevorzugt sind die Stellglieder als regulierbare Ventile ausgebildet.

Die Zufuhr der Stärkesuspension und des Enzyms in den Rührkessel erfolgt bevorzugt oben am Rührkessel. Weiterhin bevorzugt erfolgt die Zufuhr der Stärkesuspension und des Enzyms in den Rührkessel unten am Rührkessel oder weiterhin bevorzugt seitlich am Rührkessel. Die Abfuhr des abgebauten Stärkekleisters aus dem Rührkessel erfolgt bevorzugt unten am Rührkessel oder weiterhin bevorzugt seitlich am Rührkessel.

In einer bevorzugten Ausführungsform verfügt die erfindungsgemäße Anlage über eine Messvorrichtung zur Messung der Eigenschaften des abgebauten Stärkekleisters in dem Rührkessel. Bevorzugt ist die von der Messvorrichtung ermittelte Eigenschaft des abgebauten Stärkekleisters die Viskosität des abgebauten Stärkekleisters. Bevorzugt ist diese Messvorrichtung ein Prozess-online-Viskosimeter.

In einer alternativen Ausführungsform ist die Messvorrichtung bevorzugt eine Messvorrichtung zur Bestimmung des Glukosegehalts des abgebauten Stärkekleisters. Weiterhin bevorzugt verfügt die erfindungsgemäße Anlage über Messvorrichtungen zur Bestimmung des pH-Werts, der Temperatur oder der Strömungsgeschwindigkeit des abgebauten Stärkekleisters. Bevorzugt verfügt die erfindungsgemäße Anlage über mehrere Messvorrichtungen zur Bestimmung von einer oder mehreren unterschiedlichen Eigenschaften des abgebauten Stärkekleisters.

In einer weiteren bevorzugten Ausführungsform verfügt die erfindungsgemäße Anlage über Messvorrichtungen in den Zu- und Ableitungen der erfindungsgemäßen Anlage. In einer bevorzugten Ausführungsform verfügt die erfindungsgemäße Anlage über Messvorrichtungen in den Zu- und Ableitungen der Anlage und zusätzlich über Messvorrichtungen im Rührkessel. In einer bevorzugten Ausführungsform verfügt die erfindungsgemäße Anlage über Messvorrichtungen, nicht nur zur Bestimmung der Eigenschaften des abgebauten Stärkekleisters, sondern auch über Messvorrichtungen zur Bestimmung der Eigenschaften der zugeführten Stärkesuspension und des zugeführten Enzyms.

Weiterhin bevorzugt verfügt der Rührkessel der erfindungsgemäßen Anlage über Messvorrichtungen zur Bestimmung des Füllstandes im Rührkessel.

In einer bevorzugten Ausführungsform ist das Modul zur Verkleisterung der Stärkesuspension der erfindungsgemäßen Anlage so ausgebildet, dass die Stärkesuspension mit Hilfe von Dampf erhitzbar ist. Bevorzugt ist das Modul zur Verkleisterung der Stärkesuspension als ein Venturirohr ausgebildet. In einer alternativen Ausführungsform erfolgt die Verkleisterung der Stärkesuspension durch die Zufuhr von heißem Wasser oder weiterhin bevorzugt durch die Leitung der Stärkesuspension über geeignete Heizstäbe oder durch heizbare Zuleitungen.

Alternativ erfolgt die Verkleisterung der Stärkesuspension in der erfindungsgemäßen Anlage durch die Erhöhung des pH-Werts, was durch die Zugabe von geeigneter Lauge erreicht wird.

Bevorzugt verfügt die erfindungsgemäße Anlage über ein weiteres Modul zur Inaktivierung des Enzyms. Bevorzugt erfolgt die Inaktivierung des Enzyms in diesem Modul durch eine Erhöhung der Temperatur. Weiterhin bevorzugt ist das Modul zur Enzyminaktivierung so ausgebildet, dass der abgebaute Stärkekleister mit Hilfe von Dampf erhitzbar ist. In einer alternativen Ausführungsform wird die Inaktivierung des Enzyms bevorzugt über eine Veränderung des pH-Werts oder die Zugabe von Enzym-Inhibitoren erreicht. Bevorzugt ist das Modul zur Inaktivierung des Enzyms durch Signale aus der Steuereinheit der erfindungsgemäßen Anlage regulierbar.

Weitere Vorteile und Anwendungsmöglichkeiten der erfindungsgemäßen Anlage werden durch die nachfolgende schematische Abbildung erläutert.

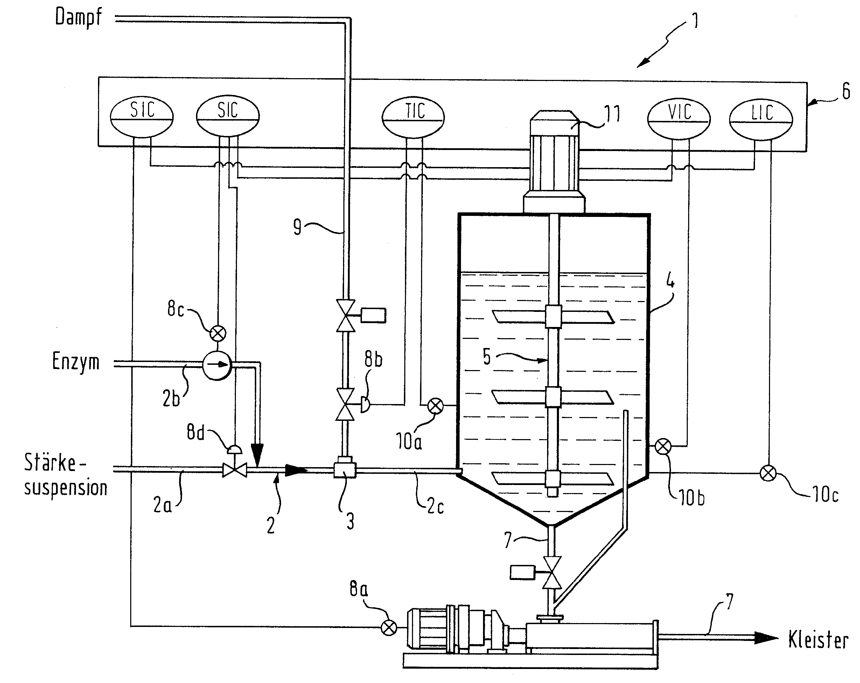

In der Abbildung ist eine Anlage (1) zur kontinuierlichen Herstellung von enzymatisch abgebautem Stärkekleister nach dem erfindungsgemäßen Verfahren dargestellt. Die Einbringung einer Stärkesuspension und eines Enzyms in einen Rührkessel (4) erfolgt über eine Zuleitung (2). Über eine Enzymzuleitung (2b) wird das Enzym in eine Stärkesuspensionszuleitung (2a) geführt, wo es sich mit der Stärkesuspension vermischt. Das Gemisch aus Enzym und Stärkesuspension wird über eine Enzym-Stärkesuspensionszuleitung (2c) in den Rührkessel (4)eingebracht. Vor dem Eintritt in den Rührkessel (4) wird das Gemisch aus Enzym und Stärkesuspension durch ein Verkleisterungsmodul (3), das als Venturirohr ausgebildet sein kann, geführt, in welchem die Stärkesuspension verkleistert wird. Der Rührkessel (4) ist mit einem Rührwerk (5) ausgestattet, um im Rührkessel einen turbulenten Zustand zu erzeugen. Das Rührwerk wird von einem Rührwerkmotor (11) angetrieben. Eine Steuereinheit (6) reguliert die Aktivität des Rührwerkmotors (11) und damit den turbulenten Zustand im Rührkessel (4). Der im Rührkessel (4) gebildete abgebaute Stärkekleister wird über eine Abführleitung (7) aus dem Rührkessel (4) abgeführt. Mit den Bezugsziffern 8a, 8b, 8c und 8d bezeichnete Stellglieder regulieren die Abführung des abgebauten Stärkekleisters (Stärkekleisterabführ-Stellglied; 8a), die Verkleisterung der Stärkesuspension (Verkleisterungs-Stellglied; 8b), die Zufuhr des Enzyms (Enzymzufuhr-Stellglied; 8c) und die Zufuhr der Stärkesuspension (Stärkesuspensionszufuhr-Stellglied; 8d). Die Stellglieder (8a, 8b, 8c, 8d) werden durch entsprechend von der Steuereinheit (6) erzeugte Signale reguliert.

Die Verkleisterung der Stärkesuspension im Verkleisterungsmodul (3) wird durch das Einbringen von Dampf über eine Dampfleitung (9) hervorgerufen. Mehrere Messvorrichtungen (10a, 10b, 10c) ermitteln unterschiedliche Eigenschaften des abgebauten Stärkekleisters im Rührkessel und leiten die ermittelten Werte an die Steuereinheit (6) weiter. Die Steuereinheit (6) verrechnet die erhaltenen Messwerte und reguliert durch entsprechende Signale die Stellglieder (8a, 8b, 8c, 8d) sowie den Motor des Rührwerks (11).

### Bezugszeichenliste

- 1: Anlage
- 2: Zuleitung
- 2a: Stärkesuspensionszuleitung
- 2b: Enzymzuleitung
- 2c: Enzym-Stärkesuspensionszuleitung
- 3: Verkleisterungsmodul
- 4: Rührkessel
- 5: Rührwerk
- 6: Steuereinheit
- 7: Stärkekleisterableitung
- 8a: Stärkekleisterabführ-Stellglied
- 8b: Verkleisterungs-Stellglied
- 8c: Enzymzufuhr-Stellglied
- 8d: Stärkesuspensionszufuhr-Stellglied
- 9: Dampfzuleitung
- 10a-c: Messvorrichtungen
- 11: Rührwerkmotor

## Patentansprüche

1. Verfahren zur kontinuierlichen Herstellung von Stärkekleister aus enzymatisch abgebauter Stärke, bei welchem einem Rührkessel (4) Stärkesuspension und Enzym zugeführt werden und gleichzeitig abgebauter Stärkekleister aus diesem Rührkessel (4) kontinuierlich abgeführt wird, wobei im abgebauten und abgeführten Kleister befindliches Enzym inaktivert wird, **dadurch gekennzeichnet,**
**dass** die Stärkesuspension und das Enzym gleichzeitig mittels einer gemeinsamen Enzym-Stärkesuspensionsleitung (2c) dem Rührkessel (4) zugeführt werden, wobei die Verkleisterung der Stärkesuspension vor dem Eintritt in den Rührkessel (4) erfolgt, und mittels eines Rührwerks (11) in dem Rührkessel (4) ein turbulenter Zustand erzeugt wird.

2. Verfahren nach Anspruch 1, bei welchem das die Stärkemoleküle abbauende Enzym eine Amylase ist.

3. Verfahren nach einem der Ansprüche 1 oder 2, bei welchem die Stärkemoleküle native oder modifizierte Stärkemoleküle sind.

4. Verfahren nach einem der Ansprüche 1 bis 3, bei welchem die Verkleisterung der Stärkesuspension vor dem Eintritt in den Rührkessel (4) in einem Venturirohr (3) erfolgt.

5. Verfahren nach einem der Ansprüche 1 bis 4, bei welchem zur Verkleisterung die Stärkesuspension mit Hilfe von Dampf erhitzt wird.

6. Verfahren nach einem der Ansprüche 1 bis 5, bei welchem die Zufuhr der Stärkesuspension und des Enzyms in den Rührkessel (4) unten, oben oder seitlich, zusammen in einer Zuleitung (2c) erfolgt.

7. Verfahren nach einem der Ansprüche 1 bis 6, bei welchem die Verweilzeit eines Stärkemoleküls im Rührkessel (4) 1 s bis zu 1 h beträgt.

8. Verfahren nach einem der Ansprüche 1 bis 7, bei welchem in dem abgeführten Stärkekleister befindliches Enzym durch Erhöhung der Temperatur inaktiviert wird.

9. Anlage zur kontinuierlichen Herstellung von Stärkekleister aus enzymatisch abgebauter Stärke und zur Durchführung des Verfahrens nach einem der Ansprüche 1 bis 8, mit
- zumindest einer Zufuhrleitung (2) zum Fördern von Enzymen und Stärkesuspension an ein Verkleisterungsmodul (3), worin eine Dampfleitung (9) endet, in der durch ein Verkleisterungs-Stellglied (8b) die Dampfzufuhr zu dem Verkleisterungsmodul (3) regulierbar ist,
- eine Enzym-Stärkesuspensionszuleitung (2c) zum Einbringen verkleisterter Stärkesuspension gemeinsam mit Enzymen in einen Rührkessel (4), der ein Rührwerk (11) zur Erzeugung eines turbulenten Strömungszustands aufweist, und ein weiteres Modul zur Inaktivierung des Enzyms vorgesehen ist,
- mindestens einer Stärkekleisterableitung (7) zur kontinuierlichen Abführung des abgebauten Stärkekleisters aus dem Rührkessel (4).

10. Anlage nach Anspruch 9, bei welcher eine Messvorrichtung zur Messung der Eigenschaften des abgebauten Stärkekleisters im Rührkessel (4) vorgesehen ist.

11. Anlage nach Anspruch 10, bei welcher das weitere Modul zur Enzyminaktivierung so ausgebildet ist, dass der abgebaute Stärkekleister mit Hilfe von Dampf erhitzbar ist.

12. Anlage nach einem der Ansprüche 9 bis 11, bei welcher der Rührkessel (4) über Schikanen oder andere Strömungsbrecher verfügt, die geeignet sind den turbulenten Zustand im Rührkessel (4) zu verstärken.

13. Anlage nach einem der Ansprüche 9 bis 12, bei der ein Stellglied (8a) zur Regulierung der Entnahme von abgebautem Stärkekleister vorgesehen ist.

14. Anlage nach einem der Ansprüche 9 bis 13, bei der eine Steuereinheit zur Regulierung des turbulenten Zustands im Rührkessel (4) vorgesehen ist.

## Claims

1. A method for continuous production of starch paste from enzymatically degraded starch, wherein starch suspension and enzyme are feeded to a stirrer vessel (4) and, at the same time, degraded starch paste is continuously removed from the stirrer vessel (4), wherein the enzyme is inactivated in the degraded and removed paste, **characterized in that** the starch suspension and the enzyme are feeded jointly to the stirrer vessel (4) via a common enzyme-starch suspension line (2c), wherein the agglutination of the starch suspension is effected before entering the stirrer vessel (4) and wherein by means of a stirrer (11) a turbulent condition is generated in the stirrer vessel (4).

2. The method in accordance with claim 1, wherein the enzyme for degrading the starch molecules is an amylase.

3. The method in accordance with one of the claims 1 or 2, wherein the starch molecules are native or modified starch molecules.

4. The method in accordance with one the claims 1 to 3, wherein the agglutination of the starch suspension is effected in a Venturi tube (3) before entering the stirrer vessel (4).

5. The method in accordance with one of the claims 1 to 4, wherein the starch suspension is heated by means of vapour for agglutination.

6. The method in accordance with one of the claims 1 to 5, wherein the feeding of the starch suspension and the enzymes jointly, to the stirrer vessel (4) is effected at a bottom, on top or lateral by means of a supply line (2c).

7. The method in accordance with one of the claims 1 to 6, wherein the dwell time for a starch molecule in the stirrer vessel (4) is 1s up to 1h.

8. The method in accordance with one of the claims 1 to 7, wherein the enzyme present in the removed starch paste is inactivated by increasing the temperature.

9. Installation for continuous production of starch paste from enzymatically degraded starch and for carrying out of the method in accordance with one of the claims 1 to 8 comprising
- at least a feed line (2) for feeding enzymes and starch suspension to an agglutination module (3), in which a vapour line (9) ends, with in which the feeding of vapour to the agglutination module (3) is controllable by means of an agglutination control element (8b),
- an enzyme-starch suspension feeding line (2c) for jointly feeding agglutinated starch suspension with enzyme to a stirrer vessel (4) having a stirrer (11) for generating turbulent conditions, and a further module for inactivating enzymes is provided,
- at least one starch paste removal line (7) for continuously removing degraded starch paste from the stirrer vessel (4).

10. Installation in accordance with claim 9, wherein a measuring device for measuring the characteristics of the degraded starch paste in the stirrer vessel (4) is provided.

11. Installation according to claim 10, wherein the further module for enzyme deactivation is designed such that degraded starch paste can be heated by vapour.

12. Installation in accordance with one of the claims 9 to 11, wherein the stirrer vessel (4) comprises baffels or other impediments to flow for increasing the turbulent condition in the stirrer vessel (4).

13. Installation in accordance with one of the claim 9 to 12, wherein a starch paste removal actuator is provided for a controlled removal of degraded starch paste.

14. Installation in accordance with one of the claims 9 to 13, wherein a control unit is provided for controlling the turbulent conditions in the stirrer vessel (4).

## Revendications

1. Procédé de fabrication continue de colle d'amidon à partir d'amidon décomposé enzymatiquement, selon lequel une suspension d'amidon et une enzyme sont introduites dans une cuve agitée (4), et une colle d'amidon décomposée est simultanément déchargée en continu de cette cuve agitée (4), l'enzyme qui se trouve dans la colle décomposée et déchargée étant inactivée, **caractérisé en ce que**
la suspension d'amidon et l'enzyme sont introduites simultanément dans la cuve agitée (4) au moyen d'une conduite d'enzyme et de suspension d'amidon commune (2c), la gélification de la suspension d'amidon ayant lieu avant l'entrée dans la cuve agitée (4) et un état turbulent étant généré dans la cuve agitée (4) au moyen d'un agitateur (11).

2. Procédé selon la revendication 1, dans lequel l'enzyme décomposant les molécules d'amidon est une amylase.

3. Procédé selon l'une quelconque des revendications 1 ou 2, dans lequel les molécules d'amidon sont des molécules d'amidon naturelles ou modifiées.

4. Procédé selon l'une quelconque des revendications 1 à 3, dans lequel la gélification de la suspension d'amidon a lieu avant l'entrée dans la cuve agitée (4) dans un tube Venturi (3).

5. Procédé selon l'une quelconque des revendications 1 à 4, dans lequel la suspension d'amidon est chauffée à l'aide de vapeur pour la gélification.

6. Procédé selon l'une quelconque des revendications 1 à 5, dans lequel l'introduction de la suspension d'amidon et de l'enzyme dans la cuve agitée (4) a lieu par le bas, par le haut ou latéralement, conjointement dans une conduite d'alimentation (2c).

7. Procédé selon l'une quelconque des revendications 1 à 6, dans lequel le temps de séjour d'une molécule d'amidon dans la cuve agitée (4) est de 1 s à 1 h.

8. Procédé selon l'une quelconque des revendications 1 à 7, dans lequel l'enzyme se trouvant dans la colle d'amidon déchargée est inactivée par élévation de la température.

9. Unité pour la fabrication continue de colle d'amidon à partir d'amidon décomposé enzymatiquement et pour la réalisation du procédé selon l'une quelconque des revendications 1 à 8, comprenant
- au moins une conduite d'alimentation (2) pour le transport d'enzymes et d'une suspension d'amidon dans un module de gélification (3), où une conduite de vapeur (9) débouche, l'introduction de vapeur dans le module de gélification (3) étant régulable par un actionneur de gélification (8b),
- une conduite d'alimentation d'enzymes et de suspension d'amidon (2c) pour l'introduction d'une suspension d'amidon gélifiée conjointement avec des enzymes dans une cuve agitée (4), qui comprend un agitateur (11) pour la génération d'un état d'écoulement turbulent, et un module supplémentaire est prévu pour l'inactivation de l'enzyme,
- au moins une conduite de déchargement de colle d'amidon (7) pour le déchargement continu de la colle d'amidon décomposée de la cuve agitée (4).

10. Unité selon la revendication 9, dans laquelle un dispositif de mesure pour la mesure des propriétés de la colle d'amidon décomposée est prévu dans la cuve agitée (4).

11. Unité selon la revendication 10, dans lequel le module supplémentaire pour l'inactivation enzymatique est configuré pour que la colle d'amidon décomposée puisse être chauffée à l'aide de vapeur.

12. Unité selon l'une quelconque des revendications 9 à 11, dans laquelle la cuve agitée (4) est munie de chicanes ou d'autres briseurs de courant, qui sont appropriés pour renforcer l'état turbulent dans la cuve agitée (4).

13. Unité selon l'une quelconque des revendications 9 à 12, dans laquelle un actionneur (8a) pour la régulation du soutirage de la colle d'amidon décomposée est prévu.

14. Unité selon l'une quelconque des revendications 9 à 13, dans laquelle une unité de commande pour la régulation de l'état turbulent dans la cuve agitée (4) est prévue.
